# EUROPEAN PATENT APPLICATION

(11) **EP 2 213 306 A1**
(43) Date of publication of application: **04.08.2010**
(21) Application number: 08831431.5
(22) Date of filing: 18.09.2008
(51) Int. Cl.: A61K 47/04, A61K 9/16, A61K 9/20, A61K 36/00

(54) **METHOD OF PRODUCING GRANULES CONTAINING MATERIAL OF NATURAL ORIGIN SUCH AS CHINESE ORTHODOX MEDICINE EXTRACT, CRUDE DRUG EXTRACT, NATURAL MATERIAL EXTRACT OR MIXTURE THEREOF AND METHOD OF PRODUCING TABLETS FROM THE GRANULES**

(30) Priority: 19.09.2007 JP 2007243046
(71) Applicant: Asahi Breweries, Ltd., Sumida-ku Tokyo 130-0001 (JP)
(72) Inventor: AZECHI, Yasutaka, Moriya-shi Ibaraki 302-0106 (JP); ISHIKAWA, Kazuyuki, Moriya-shi Ibaraki 302-0106 (JP); HIRAI, Nobuaki, Moriya-shi Ibaraki 302-0106 (JP)
(74) Representative: Feldkamp, Rainer
(86) International application number: PCT/JP2008/066883
(87) International publication number: WO 2009/038145

(57) **Abstract**

[Problems] A preparation such as granules or tablets produced by using a material of a natural origin, for example, a Chinese orthodox medicine extract, a crude drug extract or a natural material extract having been dried and powdered shows a long disintegration time, has poor elution properties and largely differs in the behavior in the patient's body after dosing from decoctions or the like, though it is a designed as inherently having a decoction, etc. as the fundamental component.

[Means for solving problems] A method of producing granules **characterized by** comprising adding to 100 parts by weight of a special calcium silicate, a material of a natural origin, which has been dispersed in a liquid or an aqueous solution containing water or an organic solvent, to give a solid content of 50 to 800 parts by weight, homogeneously mixing and dispersing by stirring the mixture, and then granulating the mixture by further stirring until the loose bulk density attains 0.3 to 0.8 g/ml.

## Description

### [Field of the art]

The present invention relates to a production method for granules containing a natural product derived substance, such as a Chinese orthodox medicine extract, a crude drug extract, or an extract derived from a natural substance, and relates to a production method for a tablet containing a Chinese orthodox medicine extract, a crude drug extract, or an extract derived from a natural substance, the tablet being produced from the granules.

### [BACKGROUND ART]

Many kinds of formulation art are used to a method of producing granules containing a natural product derived substance, such as a Chinese orthodox medicine extract, a crude drug extract, or an extract derived from a natural substance, and to a method of producing a tablet containing those extracts, the tablet being produced from the granules. However, in the conventional art, they used preliminarily dried and disintegrated powder state extract material to produce said granules and tablets. (For example, JP 2000-119190 A, JP 2001-294533 A, JP 2002-326925 A)
[Patent document 1] JP 2000-119190 A
[Patent document 2] JP 2001-294533 A
[Patent document 3] JP 2002-326925 A

### DISCLOSURE OF THE INVENTION

### PROBLEMS TO BE SOLVED BY THE INVENTION

A problem of formulation for granulated powder product (granules) and tablets using dried and powderized state natural product derived substance of a Chinese orthodox medicine extract, a crude drug extract, or an extract derived from a natural substance is that their disintegration require much time and their elution property is inferior. This means behavior of dried and powdered natural product derived substance extract inside of person's body is crucially different to behavior of formulation by a decoction and an infusion.

Since this difference stems from physicochemical property of preliminary dried and powdered Chinese orthodox medicine extract, crude drug extract, and extract derived from a natural substance, it is difficult to design a tablet equivalent, compared to formulation of decoction and an infusion, in terms of behavior of natural product derived substance inside of person's body.

In formulation of a decoction and an infusion, it is considered that a component of a natural product derived substance extract is in state of dissolution in hot water and said extract is soon integrated into digestive fluid after ingestion. In other formulation such as a tablet, however, it is reported that an effective component of a natural product derived substance extract is already powdered or crystallized since dried state extract is used, and integration into digestive fluid or water is difficult in use of dried and powdered state extract. In particular, said integration into digestive fluid or water is very few, when effective extract component having poor solubility

"Disintegrativity" in this specification means an experimentation result of disintegration test defined by "Japanese Pharmacopoeia". It also means a tablet absorbs fluid in alimentary tract and disintegrates itself after a person takes a tablet. A description of "good disintegrativity" is same meaning to "disintegrant in a short time".
"Elution" in this specification means an experimentation result of eluting test defined by Japanese Pharmacopoeia. It also means a component of a tablet is dissolved in gastrointestinal fluid, whose state is to be absorbed, after a person takes said tablet and said tablet disintegrate under fluid in stomach. A description of "good elution" is same meaning to "a component of a tablet dissolved quickly in elution testing solution which is quasi-digestive fluid".

According to the conventional art, specialty calcium silicate was used in production of tablets. This specialty calcium silicate holds liquid both oil-based and water-based and keeps powder state for its special structure. However, specialty calcium silicate powder which holds liquid has low loose bulk density, so that it cannot be used directly to produce granules and tablets. In such low bulk density, it is required to be micro-adjusted process for additional amount of water and timing of water addition, so that the production is very difficult. For example, the loose bulk density of specialty calcium silicate is approximately 0.08 mg/ml. In such low loose bulk density, a tablet will be less than 100 mg with 10 mm diameter in tableting, and cannot satisfy practical use.

The present invention provides a method of producing granules which contains high amount of natural product derived substance fed by fluid, such as a Chinese orthodox medicine extract, a crude drug extract, an extract derived from a natural substance, or a mixture of them, a tablet produced via said granules having practically sufficient size of tablet, and said granules having good elution property of effective components in said extract. And also, the present invention provides a method of producing tablets using said granules. In the present invention, the inventors considered if granulation formulation can be processed with fluid state natural product derived extract as it is fluid, of which state is before dried, before high concentrated, or before so-called soft and viscous extract. In such method to use fluid state natural product derived extract as it is fluid, the inventors consider that effective components in said fluid state extract will elute easily for extracts being not powdered (crystallized), in compared to conventional art to use dried powder extract. In such fluid state extract, poorly-soluble component may be highly dispersed in pharmaceutical formulation and may have good elution property. The inventors pursued such an art keenly, and conclude the present invention to be accomplished, being able to have good elution property even with poor solubility component in viscous and dry extracts, which is never accomplished in a marketed product. In the present invention, not only fluid state but also powder state natural product derived substance can be used to produce tablets having good elution of extract, if the natural product derived substance are prepared to dissolve or disperse in water or aqueous solution with an organic solvent.

### MEANS FOR SOLVING THE PROBLEM

The present invention provides granules which have fine fluidity and fine compression moldability, by way of adding liquid solution or dispersion liquid which includes solid component of a Chinese orthodox medicine extract, a crude drug extract, and/or an extract derived from a natural substance, to a calcium silicate which is specified as a special calcium silicate having petaloid structure, agitating the mixture of them continuously. Then, said granules are processed into a tablet.

The reason why the present invention can provides such granules is following. An extract as liquid solution or dispersion liquid is added to said special calcium silicate, and said extract penetrates into porous structure of said special calcium silicate. Also, an active constituent material in said extract is highly dispersed into a large surface area of said porous structure of special calcium silicate. Then, said active constituent material in said extract is held, in freely soluble state, in said porous structure of said special calcium silicate.
According to the present invention, even an active extract component of poor solubility can have elution property to water, if said poor soluble active extract component in a liquid solution is penetrated into a special calcium silicate.
Hereinafter, a method of agitation granulation for Chinese orthodox medicine extract, crude drug extract, an extract derived from a natural substance, and/or a mixture of them in the present invention is described.

At first, a special calcium silicate is charged into a container, and charged special calcium silicate is agitated homogeneously. Then, preliminary-prepared liquid solution or dispersion liquid which includes solid component of a Chinese orthodox medicine extract, a crude drug extract, and/or an extract derived from a natural substance, to a calcium silicate is added to said special calcium silicate, and the mixture of them is agitated in the container. As the agitation is processed, granulation is also processed on adhesive power of added Chinese orthodox medicine, crude drug medicine, and/or extract derived from a natural substance. This granulation process will be stopped if a granules has grown up to desirable size.

The time required for these processes is from 3 to 30 minutes.
In a granulation process, if amount of water in additional Chinese orthodox medicine extract, crude drug extract, or an extract derived from a natural substance is so much compared to an amount of specialty calcium silicate composition, said specialty calcium silicate cannot retain water, so that a mixture of them comes to be mud-like state and said granulation process cannot be proceeded. Therefore, if an amount of solid component is not enough, there is not sufficient cohesion in granulation and a granulation process cannot proceed appropriately. To avoid such a problem, a Chinese orthodox medicine extract, a crude drug extract, or an extract derived from a natural substance can be preliminary condensed, or a water-soluble excipient can be added to a fluid component.

The words, "agitation granulation" in the present invention, corresponds to tumbling granulation and/or wet pulverization granulation, with use of water or of liquid solution of a binder agent", of which chapter belong to "Granulation method" of "powder medicine, and tablet" in "Drug formulation", described in "Reference for The Japanese Pharmacopoeia: JP, 15th revision" (edit; The Japanese Pharmacopoeia commis □ ion, publisher; Hirokawa-Shoten Ltd., publication; July, 2006)

In this agitation granulation method, at first, a powder is fed into a container (of granulator). Then, a water or a binder-containing solution is fed into said container. After that, agitation is processed with an agitation device in the container. It is so called wet granulation.
There are many types of agitation method. For example, agitation is processed by a revolving arm on upper portion of the container and/or by a rotor blade on bottom portion of the container. In addition, said agitation can be handled by any different direction.

A vertical granulator, a high shear mixer, a high speed mixer, a planetary mixer, a planetary mixer with chopper blade, and the like are sold as an apparatus for granulation.
In the present invention, "calcium silicate" satisfies a standard of chemical name "Calcium silicate" described in "Japanese pharmaceutical excipients (Yakuji Nippo Itd., 2004) " and "Supplement To The Japanese Cosmetic Ingredients Codex (Yakuji Nippo Ltd., 1995)", and specifically indicates a petaloid crystal-structured gyrolite-type calcium silicate, which has large pore size and pore volume. More specifically, it is sold as trade name "FLORITE RE" by Tokuyama Corporation.
Chemical formula of said calcium silicate is indicated as 2CaO 3SiO2 mSiO2 nH2O (in the formula, 1<m<2, 2<n<3).

In the present invention, a Chinese orthodox medicine powder is used, solving or dispersing in a water or an ethanol aqueous solution. Said Chinese orthodox medicine powder is prepared by infusing or decocting a Chinese medicine formulation, which is described in "General companion for Chinese medicine formulation" (edited by Pharmaceutical Affairs Bureau - Ministry of Health and Welfare, published by Yakuji-jihou-sha, Ltd. (1975)), in water or in aqueous solution containing 30 wt % or less of ethanol, condensing and drying said infused or decocted solution.
Specifically, mention may be made of Chinese orthodox medicines extract powders of Sho-saiko-to, Sairei-to, Hochu-ekki-to, Saiboku-to, Gosha-jinki-gan, Kami-shoyo-san, Bakumondo-to, Hachimi-jio-gan, Dai-kenchu-to, Sho-seiryu-to, Rikkunshi-to, Toki-shakuyaku-san, Juzen-taiho-to, Kakkon-to, Saiko-keishi-to, Keishi-bukuryo-gan, Choto-san, Dai-saiko-to, Saiko-ka-ryukotsu-borei-to, Chorei-to, Unkei-to, Oren-gedoku-to, Boi-ogi-to, Gorei-san, Byakko-ka-ninjin-to, Shakuyaku-kanzo-to, Hange-byakujutsu-temma-to, Ninjin-yoei-to, Bofu-tsusho-san, Hange-shashin-to, Sho-saiko-to-ka-kikyo-sekko, Keishi-ka-jutsu-bu-to, Keigai-rengyo-to, Hange-koboku-to, Kami-kihi-to, Unsei-in, Seihai-to, Daio-kanzo-to, Jumi-haidoku-to, Toki-inshi, Shin'i-seihai-to, Toki-shigyaku-ka-goshuyu-shokyo-to, Mao-bushi-saishin-to, Otsuji-to, Kakkon-to-ka-senkyu-shin'i, Anchu-san-ryo, Shofu-san, Keishi-ka-ryukotsu-borei-to, Mao-to, Ninjin-to, Ryo-kei-jutsu-kan-to, Keishi-to, Ma-kyo-kan-seki-to, Seijo-bofu-to, Tokaku-joki-to, Sho-kenchu-to, Keishi-ka-shakuyaku-to, Kikyo-to, Shigyaku-san, Sansonin-to, Keishi-bukuryo-gan-ryo-ka-yokuinin, Ji-zuso-ippo, Shichimotsu-koka-to, Chikujo-untan-to, Shimpi-to, Goko-to, and the like. These mention do not mean limitation of the present invention, only applicable to the above-mentioned Chinese orthodox medicine powders.

In the present invention, a crude drug extract powder is also used, solving or dispersing in a water or an ethanol aqueous solution. Said crude drug extract powder is prepared by infusing or decocting at least one crude drug in water or in aqueous solution containing 30 weight percent or less of ethanol, condensing and drying said infused or decocted solution.
Specifically, mention may be made as a crude drug extract powder, such as Paeoniae Radix, Asparagi Radix, Cinnamomi Cortex, Cnidii Rhizoma, Atractylodis Lanceae Rhizoma, Poria, Moutan Cortex, Spruce, Cyperi Rhizoma, Rehmanniae Radix, Glycyrrhizae Radix, Persicae Semen, Coptidis Rhizoma, Zingiberis Rhizoma, Caryophylli Flos, Ginseng Radix, Aurantii Nobilis Pericarpium, Corydalis Tuber, Valerian, Aurantii Fructus Immaturus, Arctii Fructus, fennel, turmeric, and the like, which are crude drug medicines. These mention do not mean limitation of the present invention, only applicable to the above-mentioned crude drug extract powders.

In the present invention, a powder of extract derived from a natural substance is also used, solving or dispersing in a water or an ethanol aqueous solution. Said powder of extract derived from a natural substance is prepared by infusing or decocting a natural substance in a water or an organic solvent. Specifically, mention may be made as a powder of extract derived from a natural substance, such as Agaricus, G. pentaphyllum, Valeriana officinalis, Turmeric, Garlic, Aloe, St. John's Wort, Ginkgo leaf, and Echinacea, and the like. These mention do not mean limitation of the present invention, only applicable to the above-mentioned powder of extract derived from natural substances.

In the present invention, a Chinese orthodox medicine viscous extract or fluid extract is also used. Specifically, mention may be made as a Chinese orthodox medicine soft extract or fluid extract, such as Sho-saiko-to, Sairei-to, Hochu-ekki-to, Saiboku-to, Gosha-jinki-gan, Kami-shoyo-san, Bakumondo-to, Hachimi-jio-gan, Dai-kenchu-to, Sho-seiryu-to, Rikkunshi-to, Toki-shakuyaku-san, Juzen-taiho-to, Kakkon-to, Saiko-keishi-to, Keishi-bukuryo-gan, Choto-san, Dai-saiko-to, Saiko-ka-ryukotsu-borei-to, Chorei-to, Unkei-to, Oren-gedoku-to, Boi-ogi-to, Gorei-san, Byakko-ka-ninjin-to, Shakuyaku-kanzo-to, Hange-byakujutsu-temma-to, Ninjin-yoei-to, Bofu-tsusho-san, Hange-shashin-to, Sho-saiko-to-ka-kikyo-sekko, Keishi-ka-jutsu-bu-to, Keigai-rengyo-to, Hange-koboku-to, Kami-kihi-to, Unsei-in, Seihai-to, Daio-kanzo-to, Jumi-haidoku-to, Toki-inshi, Shin'i-seihai-to, Toki-shigyaku-ka-goshuyu-shokyo-to, Mao-bushi-saishin-to, Otsuji-to, Kakkon-to-ka-senkyu-shin'i, Anchu-san-ryo, Shofu-san, Keishi-ka-ryukotsu-borei-to, Mao-to, Mnjin-to, Ryo-kei-jutsu-kan-to, Keishi-to, Ma-kyo-kan-seki-to, Seijo-bofu-to, Tokaku-joki-to, Sho-kenchu-to, Keishi-ka-shakuyaku-to, Kikyo-to, Shigyaku-san, Sansonin-to, Keishi-bukuryo-gan-ryo-ka-yokuinin, Ji-zuso-ippo, Shichimotsu-koka-to, Chikujo-untan-to, Shimpi-to, Goko-to, and the like, which are Chinese orthodox medicines. These mention do not mean limitation of the present invention, only applicable to the above-mentioned Chinese orthodox medicine soft extract extracts or fluid extracts.

In the present invention, soft extract or fluid extract of a crude drug or a natural substance is also used. Specifically, mention may be made as a viscous extract or fluid extract of a crude drug or a natural substance, such as Agaricus, G. pentaphyllum, Ginkgo, fennel, turmeric, Siberian ginseng, Olive, asian ginseng, German Chamomile, Chinese quince, Glycyrrhizae Radix, guarana, balloon flower, Aloe, matrimony vine, Sasa veitchii, mulberry tree, Chinese cinnamon, Perilla, Ginger, St. John's Wort, Chaste tree, Bitter orange, Red pepper, Cordyceps, Chinese gutta percha, Houttuynia, jujube□Garlic, Coix seed, Safflower, Hop, Maca, adder, Hericium erinaceum, Reishi, Royal jelly, Japanese Mallotus, barrenwort, E. sagittatum, fennel, Lindera strychnifolia, Corydalis Tuber, Milk vetch, Scutellaria Root, Polygonatum sibiricum, Coptis chinensis, Polygala tenuifolia, Pueraria lobata, Curcuma zedoaria, Lycium chinense, Great yellow gentian, Panax ginseng, oriental bezoar, Schizandra chinensis, Evodia rutaecarpa, Bupleurum chinense, Asarum heterotropoides, Crataegus cuneata, Gardenia jasminoides, Cornus officinalis, Zizyphus jujuba, Dioscorea batatas, Rehmanniae Radix, Plantago asiatica, Amomum villosum, Zingiberis Rhizoma, Pheretima asiatica, Polygala senega, Ligusticum chuanxiong, Swertia japonica, Atractylodes lancea, Perillae Herba, Zizyphus jujuba, Panax japonicus, Citrus unshiu peel, Angelica sinensis, Psychotria ipecacuanha, Cuscuta chinensis, Eucommia ulmoides, Nandinae fructus, Cistanche salsa, Panax ginseng, Ophiopogon japonicus, Kanpi, Psoralea corylifolia, Ephedra sinica, silvervine, Muira Puama, Saussurea lappa, Coix lachryma-jobi, Euphoria longan, Gentianae Scabrae Radix, Velvet antler, and the like. These mention do not mean limitation of the present invention, only applicable to the above-mentioned soft extract or fluid extract of a crude drug or a natural substance.

In the present invention to produce granules containing an extract of a Chinese orthodox medicine, an extract of a crude drug, or an extract derived from a natural substance, it should be noted as the following.
Composition amount of a specialty calcium silicate is decided in comparison to drained amount or moisture amount of a fluid-state extract of said Chinese orthodox medicine, said a crude drug, or a natural substance.
When a dried and powdered extract of said Chinese orthodox medicine, said a crude drug, or a natural substance is used, solving or dispersing in a water or an ethanol aqueous solution, an amount of this fluid-state extract is decided in comparison to an amount of said specialty calcium silicate. If a moisture amount is excessive, a mixture becomes mud or paste-like state so that granulation cannot be processed. On the other hand, if a moisture amount is not enough, granulation cannot be started.
In the present invention, necessary composition amount of solid form to granulation is from 50 to 800 parts by weight, specifically from 50 to 400 parts by weight, and more specifically from 50 to 250 parts by weight, in comparison to amount of 100 parts by weight calcium silicate. In order to support this solid form composition on a specialty calcium silicate, composition amount of water or ethanol aqueous solution may be conditioned to keep appropriate fluidity of extract of said Chinese orthodox medicine, said a crude drug, or a natural substance. If a viscosity of a fluid-state extract is too thick, said fluid may be diluted accordingly.

The reason for said composition amount of solid form as the above-mentioned is that the purpose of present invention is to produce granules of which loose bulk density is from 0.3 g/ml to 0.8 g/ml. If composition amount of solid form is less than 50 parts by weight per 100 parts by weight of specialty calcium silicate, granules of such desirable loose bulk density cannot be gained. If composition amount of solid form is more than 800 parts by weight per 100 parts by weight of specialty calcium silicate, solving or dispersing fluid amount is accordingly increasing so that amount of specialty calcium silicate is relatively reduced. If an amount of a specialty calcium silicate is insufficient, a mixture becomes mud or paste-like state so that said mixture cannot become desirable granules.
In the present invention, necessary composition amount of fluid to granulation, which is except composition of solid form, is from 200 parts to 1200 parts by weight, specifically from 200 parts to 600 parts by weight, and more specifically from 200 parts to 400 parts by weight, per 100 parts by weight of specialty calcium silicate.

The reason for composition amount of fluid is as following. If an amount of fluid is less than 200 parts by weight, granules cannot be gained even if granulation is continued for long time. If an amount of fluid is more than 1200 parts by weight, a mixture becomes mud or paste-like state so that said mixture cannot become desirable granules.
In order to control additional amount of fluid component and solid component, fluid state extract of said Chinese orthodox medicine, said a crude drug, or a natural substance may be condensed or diluted in appropriate method.

In the present invention, any granulation device can be used if said device can mix a powder material appropriately. As an agitation force get strong, granulation proceeds rapidly. The agitation granulation is continued until loose bulk density of a granules become from 0.3 g/ml to 0.8 g/ml.
The resultant granules may be dried with a known drier, and fluid-bed drier or shelf drier is usually used.

After drying, the size of granules may be controlled in use of a pulverizer, granulator, and the like.
Further, an additive agent is added to said granulation product as the following.
As an excipient, there are some types of groups. One group is a type of sugar or sugar alcohol group, such aslactose, saccharose, glucose, mannitol, sorbitol, maltose, xylitol, trehalose, erythritol, and the like. Another group is a type of amylum or starch derivative group, such as cornstarch, potato starch, wheat starch, rice starch, pregelatinized starch, dextrin, carboxymethyl starch, and the like. Another group is a type of cellulose or cellulose derivative, such as crystalline cellulose, hydroxypropylcellulose, carboxymethyl cellulose, and the like. Besides, there is further other excipients, such as gum arabic, dextran, pullulan, light anhydrous silicic acid, synthetic aluminum silicate, magnesium aluminometasilicate□calcium phosphate, calcium carbonate, calcium sulfate, and the like.

Ifnecessary, the granules may be coated with water-soluble polymer. After such a coating, smell and flavor of extracts of Chinese orthodox medicines, of extract of crude drugs, or of extract derived from natural substances, may be reduced so that the granules can be easily dosed and its humidity resistance may be improved. There is no limitation of coating process and material to granules.

When a tablet is produced in use of said granules, said granules are processed on particle size regulation, and a lubricant as an additive agent is added to said granules. Then, they are mixed to produce granules for tableting. If necessary, a disintegrant may be added.
An amount of composition of disintegrant and lubricant in a tablet is decided on extract amount of Chinese orthodox medicines, of crude drugs, or derived from natural substances, on disintegrativity of a tablet, and on adherability to mortar and pestle when tableting.

As a disintegrant, it may be used of potato starch, partial alpha starch, crystalline cellulose, carboxymethyl cellulose, carboxymethylcellulose calcium, low substituted hydroxypropylcellulose, croscarmellose sodium, Sodium Starch Glycolate, cross-linked polyvinylpyrrolidone, sodium starch glycolate, and the like. Preferably, so-called super disintegrant may be used, which shows great effect in small amounts, such as croscarmellose sodium, cross-linked polyvinylpyrrolidone, sodium starch glycolate, and the like. As an amount of composition of disintegrant, it is used from 0.5 to 15 parts by weight percent, preferably from 1 to 10 parts by weight percent in a tablet.

As a lubricant, it may be used of metallic stearate (such as magnesium, and calcium), talc, hydrogenated castor oil, sodium stearyl fumarate, sucrose esters of fatty acids, and the like. As an amount of composition of lubricant, it is used from 0.1 to 5 parts by weight percent, preferably from 0.3 to 2 parts by weight percent in a tablet.
If exterior lubricant spray machine is used on tableting, it must not be including a lubricant in formulation.

Granules for tablet compression are processed on tableting machine and into a tablet. A method how to produce a tablet in use of granules is same as well-known art, so specification is omitted.
Said tablet may be further processed on pharmaceutical film coating or sugar coating. After such a coating, smell and flavor of extracts of Chinese orthodox medicines, extract of crude drugs, or extract derived from natural substances may be coated so that the tablet can be easily dosed and its humidity resistance may be improved as . tempostabile. There is no limitation of coating process and material to tablets.

### Detailed Description of the Preferred Embodiments

The invention is explained with the following embodiment.
In one embodiment, a granulator to be used is high-speed agitation granulator (trade name: VG-25, by Powrex Corporation).
Agitation rotating speed on granulation is 300 times per a minute on a bottom agitation blade device, and 3000 times per a minute on a different direction toward said bottom agitation blade device (impeller blade). This rotating speed may be controlled on fluidity of granules and process of granulation.

In drying process, a fluid-bed drier (trade name FLO-05 by Freund Corporation) is used. Air volume for drying is controlled, corresponding to fluid state of granulation portion. Temperature of said drying air is set at 80 degree centigrade.
In particle size regulation process, a granulator (trade name: COMIL-197S, by QUADRO Inc.) is used with a circular impeller and sieve screen size of 1143 µm.

In tableting process, a tableting machine (trade name: VIRGO 512 HUK, by Kikusui Seisakusyo, Ltd.) is used to press tablets by 40 rotations per minute, and a mold of 10 mm diameter R 14 is used.

### Example 1

Granules containing liquid state extract of Hachimi-jio-gan which is one of a Chinese orthodox medicine extract was produced as following.
100 parts by weight of specialty calcium silicate (Trade name: FLORITE RE) were sifted and charged into a high-speed agitation granulator. After that, 580 parts by weight of liquid state extract of Hachimi-jio-gan which included solid component of approximately 30 weight percent in said liquid state extract was added into the high-speed agitation granulator. Then, the mixture was subjected to agitation granulation for 8 minutes.
In this example, added solid component corresponds to approximately 180 parts by weight and added liquid component corresponds to approximately 400 parts by weight.

After these processed granules were dried in a fluid-bed dryer, we could obtain granules of Hachimi-jio-gan. Then, the granules were processed under particle size regulation. The granules showed good flow property in its average particle size of 300 µm and its loose bulk density of 0.65g/ml.

### Example 2

A tablet product containing liquid state extract of Hachimi-jio-gan which is one of a Chinese orthodox medicine extract was produced as follows.
9 parts by weight of cross-linked polyvinylpyrrolidone (trade name: Kollidon CL) as a disintegrant was added to 90 parts by weight of granules of example 1, and the mixture was agitated for 10 minutes by V type mixer. Then, 1 part by weight of magnesium stearate as a lubricant was added to the mixture, and further mixed and agitated for 3 minutes. This processed mixture was to use as granules for producing tablets.

Gained granules were processed into tableting. Said tableting process was set for weight of a tablet by 450 mg, and pressed on pressure by 10 kN.
According to such processed tablet, hardness of tablet is 10 kgf, disintegration time is 15 minutes, and elution rate of Cinnamic acid, which is a guiding component for poorly soluble substance of Hachimi-jio-gan extract, in water in 15 minutes is more than 85 percent.

### Example 3

Granules of Hachimi-jio-gan extract which is a Chinese orthodox medicine extract was produced as the following..
100 parts by weight of specialty calcium silicate (trade name: FLORITE RE) were sifted and charged into a high-speed agitation granulator. After that, 250 parts by weight of liquid state extract of Hachimi-jio-gan which included solid component of approximately 20 weight in said liquid state extract was added into the high-speed agitation granulator, and agitation was processed for 6 minutes.
In this example, additive amount of solid component corresponds to 50 parts by weight, and of fluid component corresponds to 200 parts by weight.

After these processed granules were dried in a fluid-bed dryer, we could obtain a granules of Hachimi-jio-gan. Then, the granules were processed under particle size regulation. The granules showed good flow property in its average particle size of 250 µm and its loose bulk density of 0.35g/ml.

### Example 4

A tablet containing of Hachimi-jio-gan extract which is a Chinese orthodox medicine extract was produced as the following..
9 parts by weight of cross-linked polyvinylpyrrolidone (trade name: Kollidon CL) as a disintegrant was added to 90 parts by weight of granulates of example 3, and the mixture was agitated for 10 minutes by V type mixer. Then, 1part by weight of magnesium stearate as a lubricant was added to the mixture, and further mixed and agitated for 3 minutes. This processed mixture was to use as granules for producing tablets.
Gained granules were processed into tableting. Said tableting process was set for weight of a tablet by 450 mg, and pressed on pressure by 10 kN.

According to such processed tablet, hardness of tablet is 10 kgf, disintegration time is 13 minutes, and elution rate of Cinnamic acid, which is a guiding component for poorly soluble substance of Hachimi-jio-gan extract, in water in 15 minutes is more than 80 percent.

### Example 5

Granules of Hachimi-jio-gan extract which is a Chinese orthodox medicine extract was produced as the following..
100 parts by weight of specialty calcium silicate (trade name: FLORITE RE) were sifted and charged into a high-speed agitation granulator. After that, Hachimi-jio-gan extract powder is solved and dispersed in purified water, adjusting so as to this aqueous solution containing an amount of solid component by 30 weight percent.
500 parts by weight of gained Hachimi-jio-gan extract solution is added to said specialty calcium silicate, and mixed and agitated for 8 minutes.

In this example, additive amount of solid component corresponds to 150 parts by weight, and of fluid component corresponds to 350 parts by weight.
After these processed granules were dried in a fluid-bed dryer, we could obtain granules of Hachimi-jio-gan. Then, the granules were processed under particle size regulation. The granules showed good flow property in its average particle size of 320 µm and its loose bulk density of 0.59g/ml.

### Example 6

A tablet containing of Hachimi-jio-gan extract which is a Chinese orthodox medicine extract was produced as the following..
9 parts by weight of cross-linked polyvinylpyrrolidone (trade name: Kollidon CL) as a disintegrant was added to 90 parts by weight of granules of example 5, and the mixture was agitated for 10 minutes by V type mixer. Then, 1part by weight of magnesium stearate as a lubricant was added to the mixture, and further mixed and agitated for 3 minutes. This processed mixture was to use as granules for producing tablets.
Gained granules were processed into tableting. Said tableting process was set for weight of a tablet by 450 mg, and pressed on pressure by 10 kN.

According to such processed tablet, hardness of tablet is 18 kgf, disintegration time is 17 minutes, and elution rate of Cinnamic acid, which is a guiding component for poorly soluble substance of Hachimi-jio-gan extract, in water in 15 minutes is more than 90 percent.

### Comparative example 1

Granules of Hachimi-jio-gan extract which is a Chinese orthodox medicine extract was produced as the following..
100 parts by weight of specialty calcium silicate (Trade name: FLORITE RE) and 240 parts by weight of powder state extract of Hachimi-jio-gan were sifted and charged into a high-speed agitation granulator. After that, 360 parts by weight of purified water was added in said agitation granulator, and agitation granulation was started. However, since said Hachimi-jio-gan extract powder was combined to water very soon, the mixture became mud or paste-like substance. Therefore, desirable granules cannot be obtained in this example.

### Comparative example 2

The inventors researched elution rate of a prior-art marketed tablet product of which starting material was Hachimi-jio-gan dried extract. In a marketed tablet product, elution rate of Cinnamic acid, which is a guiding component for poorly soluble substance of Hachimi-jio-gan extract, is as following.:
Product A: Elution rate of cinnamic acid in water in 20 minutes is 0 %.
   Elution rate of cinnamic acid in water in 30 minutes is 10 %.
Product B: Elution rate of cinnamic acid in water in 20 minutes is 0 %.
   Elution rate of cinnamic acid in water in 30 minutes is 30 %.

### Example 7

Granules of Bofu-tsusho-san extract which is a Chinese orthodox medicine extract was produced as the following..
100 parts by weight of specialty calcium silicate (trade name: FLORITE RE) were sifted and charged into a high-speed agitation granulator. After that, Bofu-tsusho-san extract powder is solved and dispersed in purified water, adjusting so as to this aqueous solution containing an amount of solid component by 25 weight percent. 600 parts by weight of gained Bofu-tsusho-san extract solution is added to said specialty calcium silicate, and mixed and agitated for 3 minutes.

In this example, additive amount of solid component corresponds to 150 parts by weight, and of fluid component corresponds to 450 parts by weight. After this processed granules were dried in a fluid-bed dryer, we could obtain granules Bofu-tsusho-san. Then, the granules were processed under particle size regulation. The granules showed good flow property in its average particle size of 300 µm and its loose bulk density of 0.55g/ml.

### Example 8

A tablet containing of Bofu-tsusho-san extract which is a Chinese orthodox medicine extract was produced as the following..
9 parts by weight of croscarmellose sodium as a disintegrant was added to 90 parts by weight of granules of example 7, and the mixture was agitated for 10 minutes by V type mixer. Then, 1part by weight of magnesium stearate as a lubricant was added to the mixture, and further mixed and agitated for 3 minutes. This processed mixture was to use as granules for producing tablets.
Gained granules were processed into tableting. Said tableting process was set for weight of a tablet by 450 mg, and pressed on pressure by 10 kN.

According to such processed tablet, hardness of tablet is 17 kgf, disintegration time is 13 minutes, and elution rate of glycyrrhizic acid, which is a guiding component of Bofu-tsusho-san, in water in 15 minutes is more than 90 percent.

### Comparative example 3

Granules of Bofu-tsusho-san extract which is a Chinese orthodox medicine extract was produced as the following..
100 parts by weight of specialty calcium silicate (Trade name: FLORITE RE) and 150 parts by weight of powder state extract of Bofu-tsusho-san were sifted and charged into a high-speed agitation granulator. After that, 450 parts by weight of purified water was added in said agitation granulator, and agitation granulation was started. However, the mixture became mud or paste-like mass substance. Therefore, desirable granules were not obtained in this example.

### Example 9

Granules of ginkgo leaf extract which is an extract derived from natural substance was produced as the following..
100 parts by weight of specialty calcium silicate (trade name: FLORITE RE) were sifted and charged into a high-speed agitation granulator. After that, 500 parts by weight of ginkgo leaf extract containing ethanol aqueous solution (purified water / ethanol = 7 / 3), adjusted so as to said solution containing an amount of solid component by 25 weight percent, were added, and mixed and agitated for 10 minutes.
In this example, additive amount of solid component corresponds to 125 parts by weight, and of fluid component corresponds to 375 parts by weight.

After these processed granules were dried in a fluid-bed dryer, we could obtain granules of ginkgo leaf extract.
Then, the granules were processed under particle size regulation. The granules powders showed good flow property in its average particle size of 280 µm and its loose bulk density of 0.55g/ml.

### Example 10

A tablet containing of ginkgo leaf extract which is is an extract derived from natural substance was produced as the following..
9 parts by weight of cross-linked polyvinylpyrrolidone (trade name: Kollidon CL) as a disintegrant was added to 90 parts by weight of granules of example 5, and the mixture was agitated for 10 minutes by V type mixer. Then, 1part by weight of magnesium stearate as a lubricant was added to the mixture, and further mixed and agitated for 3 minutes. This processed mixture was to use as granules for producing tablets.
Gained granules were processed into tableting. Said tableting process was set for weight of a tablet by 350 mg, and pressed on pressure by 8 kN.

According to such processed tablet, hardness of tablet is 10 kgf, disintegration time is 8 minutes, and elution rate of poorly soluble flavonoid (isorhamnetin) which is included in ginkgo leaf extract, in water in 60 minutes is more than 80 percent.

### Comparative example 4

The inventors researched elution rate of a prior-art marketed tablet product containing ginkgo leaf extract. In a marketed tablet product, elution rate of poorly soluble flavonoid (isorhamnetin) which is included in ginkgo leaf extract, is as following.: .
Product A: Elution rate of isorhamnetin in water in 60 minutes is 0 %.
Product B: Elution rate of isorhamnetin in water in 60 minutes is 0 %.

### Example 11

Granules of Valeriana officinalis extract which is an extract derived from natural substance was produced as the following..
100 parts by weight of specialty calcium silicate (trade name: FLORITE RE) were sifted and charged into a high-speed agitation granulator. After that, 1000 parts by weight of Valeriana officinalis extract containing water solution, adjusted so as to amount of solid component by 50 percent, were added, and mixed and agitated for a minute.

In this example, additive amount of solid component corresponds to 500 parts by weight, and of fluid component corresponds to 500 parts by weight.
After these processed granules were dried in a fluid-bed dryer, we could obtain granules of Valeriana officinalis extract.
Then, the granules were processed under particle size regulation. The granules powders showed good flow property in its average particle size of 280 µm and its loose bulk density of 0.64g/ml.

### Example 12

A tablet containing of Valeriana officinalis extract which is an extract derived from natural substance was produced as the following..
4 parts by weight of cross-linked polyvinylpyrrolidone (trade name: Kollidon CL) as a disintegrant was added to 95 parts by weight of granules of example 11, and the mixture was agitated for 10 minutes by V type mixer. Then, 1part by weight of magnesium stearate as a lubricant was added to the mixture, and further mixed and agitated for 3 minutes. This processed mixture was to use as granules for producing tablets.

Gained granules were processed into tableting. Said tableting process was set for weight of a tablet by 380 mg, and pressed on pressure by 10 kN.
According to such processed tablet, hardness of tablet is 14 kgf, and disintegration time is 12 minutes.

### Comparative example 5

Granules of Valeriana officinalis extract which is an extract derived from natural substance was produced as the following..
100 parts by weight of specialty calcium silicate (Trade name: FLORITE RE) were sifted and charged into a high-speed agitation granulator. Then, 500 parts by weight of extract of Valeriana officinalis extract were charged into said granulator. After that, a mixture of them were mixed and agitated for a minute, and then, 500 parts by weight of purified water was added into said agitation granulator. When agitation granulation was started, the mixture became mud or paste-like substance. Also, said mud or paste-like substance do not obtain good fluidity, even after drying.

### Example 13

Granules of St. John's Wort extract which is an extract derived from natural substance was produced as the following.
100 parts by weight of specialty calcium silicate (trade name: FLORITE RE) were sifted and charged into a high-speed agitation granulator. After that, 550 parts by weight of St. John's Wort extract containing ethanol aqueous solution (purified water/ethanol = 1/1), adjusted so as to said solution containing an amount of solid component by 30 weight percent, were added, and mixed and agitated for 10 minutes.

In this example, additive amount of solid component corresponds to 165 parts by weight, and of fluid component corresponds to 385 parts by weight.
After these processed granules were dried in a fluid-bed dryer, we could obtain granules of St. John's Wort extract. Then, the granules were processed under particle size regulation. The granules showed good flow property in its average particle size of 260 µm and its loose bulk density of 0.39g/ml.

### Example 14

A tablet containing St. John's Wort extract which is an extract derived from natural substance was produced as the following..
4 parts by weight of Sodium Starch Glycolate as a disintegrant was added to 95 parts by weight of granules of example 13, and the mixture was agitated for 10 minutes by V type mixer. Then, 1part by weight of magnesium stearate as a lubricant was added to the mixture, and further mixed and agitated for 3 minutes. This processed mixture was to use as granules for producing tablets.

Gained granules were processed into tableting. Said tableting process was set for weight of a tablet by 380 mg, and pressed on pressure by 8 kN.
According to such processed tablet, hardness of tablet is 16 kgf, disintegration time is 11 minutes, and elution rate of hyperforin, which is a guiding component for St. John's Wort extract, in water in 60 minutes is more than 50 percent.

### Comparative example 6

The inventors researched elution rate of a prior-art marketed tablet product containing St. John's Wort extract. In a marketed tablet product, elution rate of poorly soluble hyperforin which is included in St. John's Wort extract, is as follows.:
Product A: Elution rate of hyperforin in water in 60 minutes is 0 %.

## Claims

1. A method of producing granules, comprising the steps of:
preliminary solving or dispersing a natural product derived substance in water or aqueous solution with organic solvent to form a solution thereof;
adding said solution to 100 parts by weight of specialty calcium silicate to form a mixture, whereby a solid component of said natural products derived substance in said solution is from 50 to 800 parts by weight;
uniformly dispersing and agitating the mixture;
agitating and granulating said resultant mixture, until loose bulk density of granules produced is from 0.3g to 0.8g/ml.

2. A method of producing granules, comprising the steps of:
preliminary solving or dispersing natural products derived substance in water or aqueous solution with organic solvent to form a solution thereof;
adding said solution to 100 parts by weight of specialty calcium silicate to form a mixture, whereby a fluid component of said natural products derived substance in said solution is from 200 to 1200 parts by weight;
uniformly dispersing and agitating the mixture;
agitating and granulating said mixture, until loose bulk density of granules produced is from 0.3g to 0.8g/ml.

3. A method of producing granules, according to claim 1 and 2, wherein said natural product derived substance is a Chinese orthodox medicine extract.

4. A method of producing granules, according to claim 1 and 2, wherein said natural product derived substance is a crude drug extract.

5. A method of producing granules, according to claim 1 and 2, wherein said natural product derived substance is an extract derived from natural substance.

6. A method of producing granules, according to claim 1 and 2, wherein said specialty calcium silicate is gyrolite-type calcium silicate.

7. A method of producing a tablet, adding a disintegrant into granules according to claim 1 or 2, mixing a resultant, then tableting said resultant.

8. A method of producing a tablet, adding a disintegrant and a lubricant into granules according to claim 1 or 2, mixing a resultant, then tableting said resultant.
